# EUROPEAN PATENT APPLICATION

(11) **EP 4 190 259 A1**
(43) Date of publication of application: **07.06.2023**
(21) Application number: 22210508.2
(22) Date of filing: 30.11.2022
(51) Int. Cl.: A61B 18/14, A61B 90/00, G06N 20/00

(54) **ACTIVE CONTROL OF SURGICAL SMOKE EVACUATION SYSTEMS**

(30) Priority: 01.12.2021 US 202163284729 P; 25.10.2022 US 202217973131
(71) Applicant: Covidien LP, Mansfield, MA 02048 (US)
(72) Inventor: VADALI, Manoj Kumar, K V S, 502032 Hyderabad (IN); RAI, Sarita, 500073 Hyderabad (IN)
(74) Representative: Maschio, Antonio

(57) **Abstract**

A surgical smoke evacuation system includes an imaging device configured to capture an image of a surgical site, a smoke evacuator in communication with the imaging device and including a suction generator configured to create a vacuum pressure, an electrosurgical pencil including a nozzle, a suction conduit coupling the nozzle to the smoke evacuator, a processor, and a memory. The memory includes instructions stored thereon which, when executed by the processor, cause the surgical smoke evacuation system to: identify a feature in the captured image of the surgical site; classify an amount of smoke in the image using a machine learning network based on the identified feature; and dynamically adjust the vacuum pressure generated by the smoke evacuator based on the classified amount of smoke in the image.

## Description

### CROSS-REFERENCE TO RELATED APPLICATIONS

The present application claims the benefit of and priority to U.S. Provisional Patent Application Serial No. 63/284,729, filed on December 1, 2021, the entire content of which being hereby incorporated by reference.

### TECHNICAL FIELD

The disclosure relates to surgical devices. More specifically, the disclosure relates to active control of surgical smoke evacuation systems.

### BACKGROUND OF RELATED ART

Surgical smoke evacuation systems are typically used with electrosurgical (ES) pencils during surgery for evacuating smoke generated during use of the ES pencil. The ES pencil is generally used for cutting tissue and/or for coagulating blood vessels. An ES pencil usually includes a handpiece into which electrodes of various shapes and sizes may be placed. The ES pencil is coupled to an ES generator, such as Medtronic's Valleylab^{™} LS10 or FT10 generator, which supplies the electrode with a high frequency, typically radio frequency (RF) alternating current. The ES generator may supply various waveforms suitable for achieving various surgical effects, such as cutting, coagulating, blending, spraying, fulgurating, and the like.

While using an ES pencil, smoke is often generated. An effective way to evacuate surgical smoke is to use an ES pencil with an integrated smoke evacuation nozzle in conjunction with a suction device and an ultra-low penetration air (ULPA) filter. Conventional ES pencils rely on smoke evacuation shrouds attached to the ES pencil, which suction the smoke away via a suction device. Smoke shrouds are available either as an integrated part of the ES pencil or as a separate shroud attached to the ES pencil. A smoke nozzle, situated near the pencil's electrode, draws the smoke plume into and through the pencil's body, through a long flexible hose, and finally into a powered suction device outside of the surgical field.

During a surgical procedure, clinicians often have to spend time identifying the appropriate speed of the smoke evacuator and when to enable and disable the surgical smoke evacuation system.

### SUMMARY

The disclosure includes multiple embodiments, each of which includes multiple aspects. Various aspects of the embodiments are interchangeable among the disclosed aspects.

In accordance with embodiments of the disclosure, a surgical smoke evacuation system includes an imaging device configured to capture an image of a surgical site and a smoke evacuator in communication with the imaging device. The system also includes a suction generator configured to create a vacuum pressure and an electrosurgical pencil having a nozzle and a suction conduit coupling the nozzle to the smoke evacuator. The system further includes a processor and a memory having instructions stored thereon which, when executed by the processor, cause the surgical smoke evacuation system to: identify a feature in the captured image of the surgical site; classify an amount of smoke in the image using a machine learning network based on the identified feature; and dynamically control the smoke evacuator based on the classified amount of smoke in the image.

In an aspect of the disclosure, the feature may include a type of tissue in the image.

In another aspect of the disclosure, the type of tissue in the image may include fatty tissue and/or normal tissue.

In yet another aspect of the disclosure, the feature may include a rate of change of area of smoke spread in the captured image.

In a further aspect of the disclosure, the feature may include a number of locations of smoke generation in the captured image.

In yet a further aspect of the disclosure, the feature may include an amount of smoke in the captured image.

In an aspect of the disclosure, the machine learning network may include a support vector machine, a hidden Markov model, and/or a convolutional neural network.

In another aspect of the disclosure, the imaging device may communicate wirelessly with the smoke evacuator.

In yet another aspect of the disclosure, the instructions, when executed, may further cause the smoke evacuation system to receive a signal indicating whether energy is being applied to tissue in the captured image, by the electrosurgical pencil and increase a sampling rate of the imaging device in response to the energy being applied to the tissue in the captured image.

In a further aspect of the disclosure, the instructions, when executed, may further cause the smoke evacuation system to decrease the sampling rate of the imaging device in response to the energy not being applied to the tissue in the captured image.

In accordance with embodiments of the disclosure, a computer-implemented method for controlling a surgical smoke evacuation system is presented. The method includes capturing an image of a surgical site by an imaging device, identifying a feature in the captured image of the surgical site, classifying an amount of smoke in the image using a machine learning network based on the identified feature, and dynamically adjusting a vacuum pressure generated by a smoke evacuator based on the classified amount of smoke in the image.

In an aspect of the disclosure, the feature may include a type of tissue in the image.

In another aspect of the disclosure, the type of tissue in the image may include fatty tissue and/or normal tissue.

In yet another aspect of the disclosure, the feature may include a rate of change of area of smoke spread in the captured image.

In a further aspect of the disclosure, the feature may include a number of locations of smoke generation in the captured image.

In yet a further aspect of the disclosure, the feature may include an amount of smoke in the captured image.

In an aspect of the disclosure, the machine learning network may include a support vector machine, a hidden Markov model, and/or a convolutional neural network.

In another aspect of the disclosure, the imaging device may communicate wirelessly with the smoke evacuator.

In an aspect of the disclosure, the method may further include receiving a signal indicating whether energy is being applied to tissue in the captured image, by an electrosurgical pencil and increase a sampling rate of the imaging device in response to the energy being applied to the tissue in the captured image.

In accordance with embodiments of the disclosure, a non-transitory computer-readable medium storing instructions which, when executed by a processor, cause the processor to perform a method for controlling a surgical smoke evacuation system is presented. The method includes capturing an image of a surgical site, identifying a feature in the captured image of the surgical site, classifying an amount of smoke in the image using a machine learning network based on the identified feature, and dynamically adjusting a vacuum pressure generated by a smoke evacuator based on the classified amount of smoke in the image.

### BRIEF DESCRIPTION OF THE DRAWINGS

Aspects of the disclosure are described herein with reference to the accompanying drawings, wherein:
FIG. 1 is a front view of a surgical smoke evacuation system, in accordance with the disclosure;
FIG. 2 is a perspective view of a smoke evacuator and an electrosurgical (ES) pencil of the surgical smoke evacuation system of FIG. 1, in accordance with the disclosure;
FIG. 3 is a flow diagram of a machine learning network of the computer-controlled method for controlling the surgical smoke evacuation system of FIG. 1;
FIG. 4 is a flow chart of a computer-controlled method for controlling a surgical smoke evacuation system of FIG. 1;
FIG. 5A is an image of a surgical site showing no smoke;
FIG. 5B is an image of the surgical site showing low amounts of smoke;
FIG. 5C is an image of the surgical site showing moderate amounts of smoke; and
FIG. 5D is an image of the surgical site showing high amounts of smoke.

### DETAILED DESCRIPTION OF ASPECTS

Embodiments of the disclosure are now described in detail with reference to the drawings in which like reference numerals designate identical or corresponding elements in each of the drawings. The aspects may be combined in any manner consistent with the functionality of the apparatus and/or method disclosed herein. As used herein, the term "clinician" refers to a doctor, a nurse or any other care provider and may include support personnel. Throughout this description, the term "proximal" will refer to the portion of the device or component thereof that is closer to the clinician and the term "distal" will refer to the portion of the device or component thereof that is farther from the clinician. The terms "substantially equal to" or "substantially the same" denote values that are within ±5% of each other. Additionally, in the drawings and in the description that follows, terms such as front, rear, upper, lower, top, bottom, and similar directional terms are used simply for convenience of description and are not intended to limit the disclosure. In the following description, well-known functions or constructions are not described in detail to avoid obscuring the disclosure in unnecessary detail.

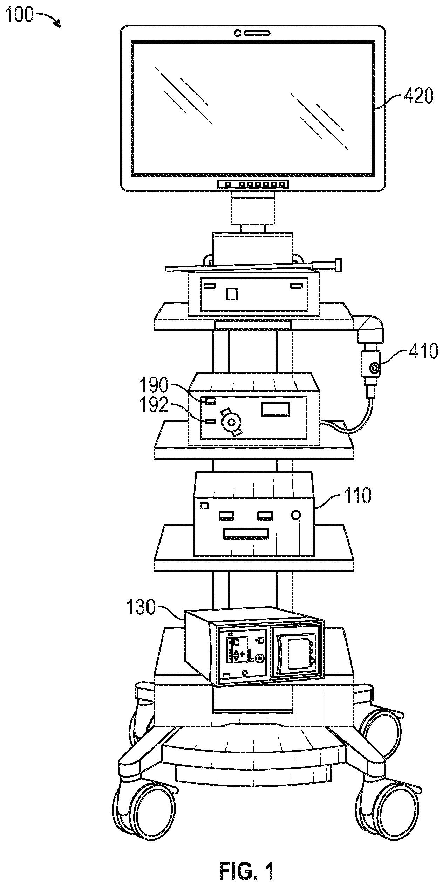

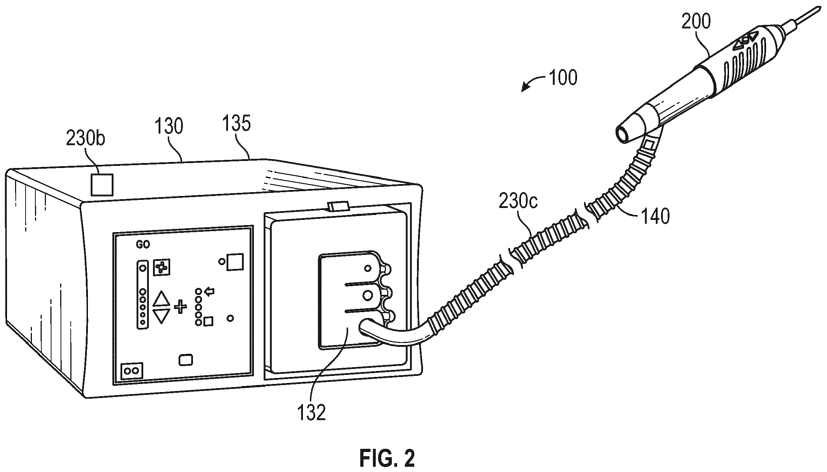

With reference to FIGS. 1 and 2, a surgical smoke evacuation system 100 is shown. The surgical smoke evacuation system 100 generally includes a smoke evacuator 130, an ES pencil 200, a suction conduit 140 connecting the ES pencil 200 to the smoke evacuator 130, and an imaging device 410 (FIG. 2) configured to capture images of a surgical site. The ES pencil 200 is also coupled to an ES generator 110, which may be any suitable ES generator configured to supply radio frequency energy to the ES pencil 200.

The smoke evacuator 130 includes a suction generator 135 that creates negative pressure having a set vacuum force for removing smoke during a surgical operation. The suction conduit 140 is connected to the ES pencil 200 at its distal end and to an inlet port 132 of the smoke evacuator 130 at its proximal end. The suction generator 135 may include one or more fans to create the negative pressure enabling smoke removal from a surgical site.

The surgical smoke evacuation system 100 also includes a processor 190 and a memory 192. Instructions may be executed by the processor 190, which may include one or more digital signal processors (DSPs), general-purpose microprocessors, application-specific integrated circuits (ASICs), field programmable logic arrays (FPGAs), or other equivalent integrated or discrete logic circuitry. Accordingly, the term "processor" as used herein may refer to any of the foregoing structures or any other physical structure suitable for implementation of the described techniques. Also, the techniques could be fully implemented in one or more circuits or logic elements. It is contemplated that the processor 190 and memory 192 may also be located in the imaging device 410, the smoke evacuator 130, the ES pencil 200, and/or in a remote computer system.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

ES pencil 200 may be used for cutting tissue and/or for coagulating blood vessels. The ES pencil 200 usually includes a handpiece into which electrodes of various shapes and sizes may be placed. The ES pencil 200 may include a nozzle 212 situated near the ES pencil's 200 electrode 214, draws the smoke plume into and through the ES pencil's body, through the suction conduit 140, and finally into the smoke evacuator 130.

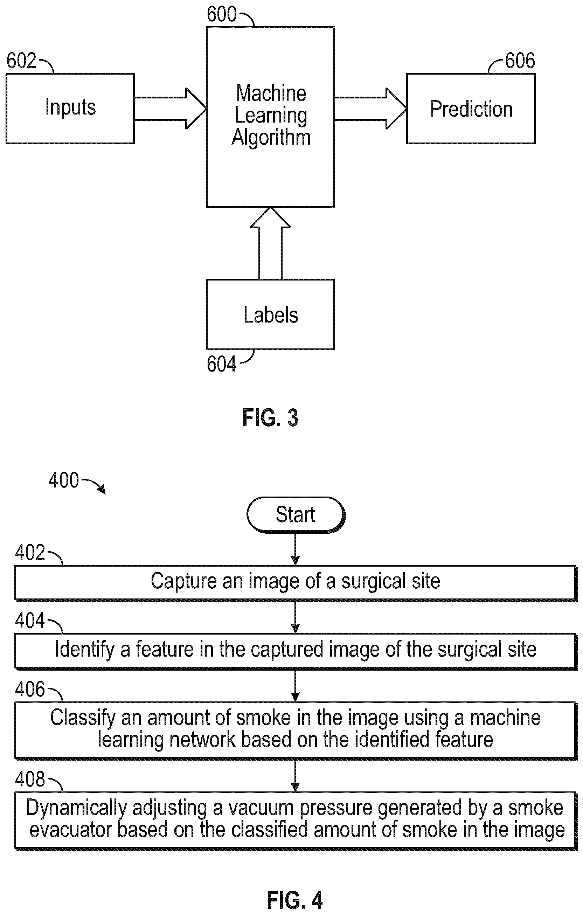

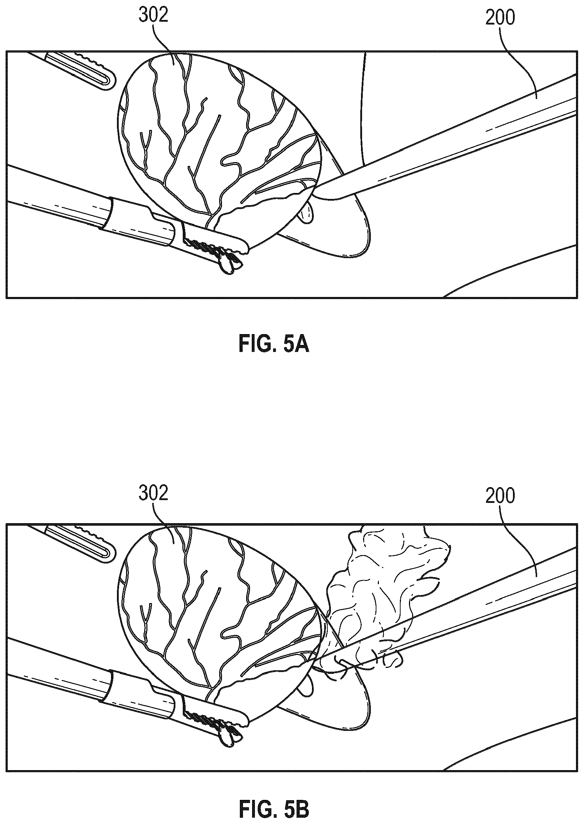

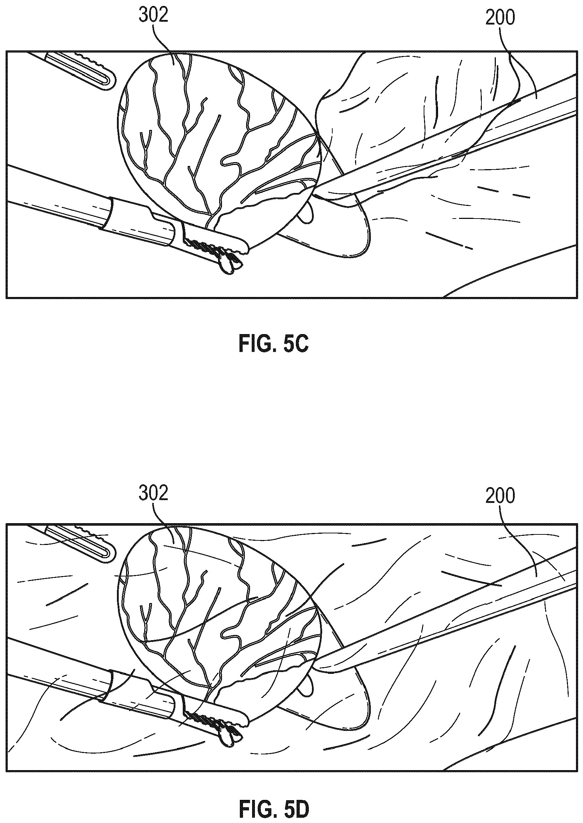

The imaging device 410 is configured to capture real-time images of the surgical site for viewing by the clinician and for processing by a machine learning network 600 (FIG. 3), which may be executed, for example, by processor 190 and/or by a remote device. The machine learning network 600 is configured to classify the real-time images based on the amount of smoke in the images or based on other features which indicate that there will be smoke produced during application of energy to tissue. In embodiments, an image with no smoke (FIG. 5A) may be classified as "0=no smoke"; an image with relatively little smoke (FIG. 5B) may be classified as "1=low smoke"; an image with moderate smoke (FIG. 5C) may be classified as "2=moderate smoke"; and an image with a relatively high amount of smoke (FIG. 5D) may be classified as "3=high smoke." It is contemplated that other classification schemes may be used. The imaging device 410 may communicate with the smoke evacuator 130 in a wired or a wireless manner, e.g., Bluetooth, WiFi, Interlink cable, optical, or any other suitable means.

The surgical smoke evacuation system 100 may include a display 420 configured for displaying information such as real-time images of the surgical site. The display 420 may also display warnings and/or settings of the surgical smoke evacuation system 100.

With reference to FIG. 3, the processor 190 may include a machine learning network 600 configured to make these evaluations. The processor 190 may use machine learning to predict 606 an amount of smoke based on the captured images 602. Machine learning may include a convolutional neural network (CNN) and/or a state variant machine (SVM). The CNN may be trained on previous images of tissue and/or smoke. The machine learning network 600 may be trained using supervised training and/or unsupervised training. In aspects, the machine learning network 600 may classify the type of tissue in the captured image(s), and/or may classify the amount of smoke in the captured image(s).

Referring to FIG. 4, there is shown a flow chart of an exemplary computer-implemented method 400 for controlling the surgical smoke evacuation system 100 in accordance with aspects of the present disclosure. Although the steps of FIG. 4 are shown in a particular order, the steps need not all be performed in the specified order, and certain steps can be performed in another order. For simplicity, FIG. 4 will be described below, with the processor 190 performing the operations. However, in various aspects, the operations of FIG. 4 may be performed in part by the processor 190 of FIG. 1 and in part by another device, such as a remote server. These variations are contemplated to be within the scope of the present disclosure.

During surgery, smoke may be generated while applying RF energy using the ES pencil 200 to cut and/or coagulate tissue. The disclosed technology enables the dynamic and automatic control of the surgical smoke evacuation system 100 to efficiently and effectively reduce smoke.

Initially, at step 402, during a surgical procedure, the imaging device 410 captures an image (or a series of images, e.g., video) of a surgical site (FIGS. 5A-D). The captured image (FIGS. 5A-D) may include tissue 302, and/or surgical instruments/tools (e.g., ES pencil 200). The captured image may include various amounts of smoke generated when the ES pencil 200 is cutting tissue and/or coagulating blood vessels.

At step 404, the processor 190 identifies a feature in the captured image of the surgical site. In aspects, the feature may include a type of tissue in the image. When cutting/coagulating fatty tissue, a large amount of smoke may be generated relative to non-fatty tissue. Fatty tissue generally appears to be whiteish when compared to normal non-fatty tissue. The tissue type, e.g., fatty tissue, may be identified by an image processing algorithm (e.g., a classification algorithm and/or a segmentation algorithm). Based on the tissue identification, the processor 190 may determine the vacuum pressure generated by the smoke evacuator 130 prior to the appearance of smoke in the captured image to minimize the smoke accumulation in the surgical cavity, even prior to energy activation of the ES pencil 200.

In another aspect, the feature may include a rate of change of area of smoke spread in the captured image. When the area of smoke spread is increasing at faster rate, the smoke evacuator 130 is set to a higher vacuum pressure. In aspects, the processor 190 may identify the "area of smoke spread" in each frame of the captured images (e.g., video) and compare this identified area in each new frame with the previous frame. The identified rate of change of area of smoke spread in the captured images provides information about the amount of area occupied by smoke and the rate at which the smoke is generating and spreading.

In another aspect, the feature may include a number of locations of smoke generation in the captured image. When smoke is generated from multiple locations in the surgical site, the cumulative area of smoke spread may be determined by adding the area of spread of each smoke location. In aspects, the processor 190 may identify a number of areas of smoke generation in the image to be classified by the machine learning network 600 for use in determining the vacuum pressure generated by the smoke evacuator 130. Thus, if the number of smoke generation locations is relatively large in quantity (e.g., about 4 locations), then the processor 190 may dynamically increase determining the vacuum pressure generated by the smoke evacuator 130. If the number of smoke generation locations is relatively small in quantity (e.g., about one location), then the processor 190 may dynamically decrease determining the vacuum pressure generated by the smoke evacuator 130.

In another aspect, the feature may include an amount of smoke in the captured image. The processor 190 may use an image processing algorithm such as image segmentation to determine the amount of smoke in the image. FIGS. 5A-D show captured images with progressively larger amounts of smoke in the image.

At step 406, the processor uses a machine learning network 600 to classify an amount of smoke in the image based on the identified feature. In aspects, the machine learning network may include one or more of a support vector machine, a hidden Markov model, or a CNN. Thus, if the captured image contained a high amount of smoke (FIG. 5D), then the machine learning network may classify the image as "3=high smoke." In aspects, images may be classified based on: the rate of change of area of smoke spread, the number of locations of smoke generation, the type of tissue that is in contact with monopolar/bipolar (e.g., fat tissue white gives more smoke), energy application (e.g., power on generators), and/or the smoke evacuator mode of operation (e.g., on/off and/or speed level ).

At step 408, the processor 190 dynamically adjusts the vacuum pressure generated by the smoke evacuator 130, based on the classification of the captured image. If the machine learning network classified the image as "3=high smoke," then the processor 190 would dynamically increase the vacuum pressure generated by the smoke evacuator 130, to reduce the high level of smoke.

A benefit of the disclosed technology is that it provides closed-loop feedback between the smoke evacuator and the area with smoke. The processor 190 may, based on the rate of spread of area of the smoke, determines the desired vacuum pressure and dynamically adjusts the vacuum pressure generated by the smoke evacuator 130. The processor 190 may then sense the rate of smoke reduction based on new images being captured and classified. Based on the rate of reduction, the processor 190 may then dynamically adjust the vacuum pressure generated by the smoke evacuator 130.

In aspects, the processor 190 may receive a signal indicating whether energy is being applied to tissue in the captured image, by the ES pencil 200. In a case where the energy is being applied to the tissue in the captured image, the processor 190 may increase a sampling rate of the imaging device. In a case where energy is not being applied to the tissue in the captured image, decrease the sampling rate of the imaging device. Thus, when the energy is not applied to the tissue, sampling rate of the image capture by the imaging device 410 is low, and once the energy is applied the sampling rate would be set as high. This feature allows for reducing the computational load on the system and improves the overall performance.

It will be understood that various modifications may be made to the aspects of the presently disclosed surgical smoke evacuation systems. Therefore, the above description should not be construed as limiting but merely as exemplifications of aspects. Those skilled in the art will envision other modifications within the scope and spirit of the disclosure.

The invention may be described by reference to the following numbered paragraphs:-
1. A surgical smoke evacuation system, comprising:
   an imaging device configured to capture an image of a surgical site;
   a smoke evacuator in communication with the imaging device and including a suction generator configured to create a vacuum pressure;
   an electrosurgical pencil including a nozzle;
   a suction conduit coupling the nozzle to the smoke evacuator;
   a processor; and
   a memory, including instructions stored thereon which, when executed by the processor, cause the surgical smoke evacuation system to:
      identify a feature in the captured image of the surgical site;
      classify an amount of smoke in the image using a machine learning network based on the identified feature; and
      dynamically adjust the vacuum pressure generated by the smoke evacuator based on the classified amount of smoke in the image.
2. The smoke evacuation system according to paragraph 1, wherein the feature includes a type of tissue in the image.
3. The smoke evacuation system according to paragraph 2, wherein the type of tissue in the image includes at least one of fatty tissue or normal tissue.
4. The smoke evacuation system according to paragraph 1, wherein the feature includes a rate of change of area of smoke spread in the captured image.
5. The smoke evacuation system according to paragraph 1, wherein the feature includes a number of locations of smoke generation in the captured image.
6. The smoke evacuation system according to paragraph 1, wherein the feature includes an amount of smoke in the captured image.
7. The smoke evacuation system according to paragraph 1, wherein the machine learning network includes at least one of a support vector machine, a hidden Markov model, or a convolutional neural network.
8. The smoke evacuation system according to paragraph 1, wherein the imaging device communicates wirelessly with the smoke evacuator.
9. The smoke evacuation system according to paragraph 1, wherein the instructions, when executed, further cause the smoke evacuation system to:
   receive a signal indicating whether energy is being applied to tissue in the captured image, by the electrosurgical pencil; and
   increase a sampling rate of the imaging device in response to the energy being applied to the tissue in the captured image.
10. The smoke evacuation system according to paragraph 9, wherein the instructions, when executed, further cause the smoke evacuation system to:
   decrease the sampling rate of the imaging device in response to the energy not being applied to the tissue in the captured image.
11. A computer-implemented method for controlling a surgical smoke evacuation system, comprising:
   capturing an image of a surgical site, by an imaging device;
   identifying a feature in the captured image of the surgical site;
   classifying an amount of smoke in the image using a machine learning network based on the identified feature; and
   dynamically adjusting a vacuum pressure generated by a smoke evacuator based on the classified amount of smoke in the image.
12. The computer-implemented method according to paragraph 11, wherein the feature includes a type of tissue in the image.
13. The computer-implemented method according to paragraph 12, wherein the type of tissue in the image includes at least one of fatty tissue or normal tissue.
14. The computer-implemented method according to paragraph 11, wherein the feature includes a rate of change of area of smoke spread in the captured image.
15. The computer-implemented method according to paragraph 11, wherein the feature includes a number of locations of smoke generation in the captured image.
16. The computer-implemented method according to paragraph 11, wherein the feature includes an amount of smoke in the captured image.
17. The computer-implemented method according to paragraph 11, wherein the machine learning network includes at least one of a support vector machine, a hidden Markov model, or a convolutional neural network.
18. The computer-implemented method according to paragraph 11, wherein the imaging device communicates wirelessly with the smoke evacuator.
19. The computer-implemented method according to paragraph 11, further comprising:
   receiving a signal indicating whether energy is being applied to tissue in the captured image, by an electrosurgical pencil; and
   increase a sampling rate of the imaging device in response to the energy being applied to the tissue in the captured image.
20. A non-transitory computer-readable medium storing instructions which, when executed by a processor, cause the processor to perform a method for controlling a surgical smoke evacuation system, comprising:
   capturing an image of a surgical site;
   identifying a feature in the captured image of the surgical site;
   classifying an amount of smoke in the image using a machine learning network based on the identified feature; and
   dynamically adjusting a vacuum pressure generated by a smoke evacuator based on the classified amount of smoke in the image.

## Claims

1. A surgical smoke evacuation system, comprising:
an imaging device configured to capture an image of a surgical site;
a smoke evacuator in communication with the imaging device and including a suction generator configured to create a vacuum pressure;
an electrosurgical pencil including a nozzle;
a suction conduit coupling the nozzle to the smoke evacuator;
a processor; and
a memory, including instructions stored thereon which, when executed by the processor, cause the surgical smoke evacuation system to:
identify a feature in the captured image of the surgical site;
classify an amount of smoke in the image using a machine learning network based on the identified feature; and
dynamically adjust the vacuum pressure generated by the smoke evacuator based on the classified amount of smoke in the image.

2. The smoke evacuation system according to claim 1, wherein the feature includes a type of tissue in the image.

3. The smoke evacuation system according to claim 2, wherein the type of tissue in the image includes at least one of fatty tissue or normal tissue.

4. The smoke evacuation system according to any preceding claim, wherein the feature includes a rate of change of area of smoke spread in the captured image.

5. The smoke evacuation system according to any preceding claim, wherein the feature includes a number of locations of smoke generation in the captured image; and/or wherein the feature includes an amount of smoke in the captured image.

6. The smoke evacuation system according to any preceding claim, wherein the machine learning network includes at least one of a support vector machine, a hidden Markov model, or a convolutional neural network; and/or wherein the imaging device communicates wirelessly with the smoke evacuator.

7. The smoke evacuation system according to any preceding claim, wherein the instructions, when executed, further cause the smoke evacuation system to:
receive a signal indicating whether energy is being applied to tissue in the captured image, by the electrosurgical pencil; and
increase a sampling rate of the imaging device in response to the energy being applied to the tissue in the captured image; preferably wherein the instructions, when executed, further cause the smoke evacuation system to:
decrease the sampling rate of the imaging device in response to the energy not being applied to the tissue in the captured image.

8. A computer-implemented method for controlling a surgical smoke evacuation system, comprising:
capturing an image of a surgical site, by an imaging device;
identifying a feature in the captured image of the surgical site;
classifying an amount of smoke in the image using a machine learning network based on the identified feature; and
dynamically adjusting a vacuum pressure generated by a smoke evacuator based on the classified amount of smoke in the image.

9. The computer-implemented method according to claim 8, wherein the feature includes a type of tissue in the image; preferably wherein the type of tissue in the image includes at least one of fatty tissue or normal tissue.

10. The computer-implemented method according to claim 8 or claim 9, wherein the feature includes a rate of change of area of smoke spread in the captured image.

11. The computer-implemented method according to any of claims 8 to 10 wherein the feature includes a number of locations of smoke generation in the captured image; and/or wherein the feature includes an amount of smoke in the captured image.

12. The computer-implemented method according to any of claims 8 to 11, wherein the machine learning network includes at least one of a support vector machine, a hidden Markov model, or a convolutional neural network' preferably wherein the imaging device communicates wirelessly with the smoke evacuator.

13. The computer-implemented method according to any of claims 8 to 12, further comprising:
receiving a signal indicating whether energy is being applied to tissue in the captured image, by an electrosurgical pencil; and
increase a sampling rate of the imaging device in response to the energy being applied to the tissue in the captured image.

14. A non-transitory computer-readable medium storing instructions which, when executed by a processor, cause the processor to perform a method for controlling a surgical smoke evacuation system, comprising:
capturing an image of a surgical site;
identifying a feature in the captured image of the surgical site;
classifying an amount of smoke in the image using a machine learning network based on the identified feature; and
dynamically adjusting a vacuum pressure generated by a smoke evacuator based on the classified amount of smoke in the image.
